# EUROPEAN PATENT APPLICATION

(11) **EP 4 588 419 A1**
(43) Date of publication of application: **23.07.2025**
(21) Application number: 24152655.7
(22) Date of filing: 18.01.2024
(51) Int. Cl.: A61B 1/00, A61B 1/04, A61B 1/06

(54) **DATA PROCESSING DEVICE FOR A MEDICAL OBSERVATION APPARATUS, COMPUTER-IMPLEMENTED METHOD AND METHOD FOR OBSERVING AN OBJECT UNDER NON-WHITE LIGHT ILLUMINATION**

(71) Applicant: Leica Instruments (Singapore) Pte Ltd, Singapore 608924 (SG)
(72) Inventor: THEMELIS, George, 608924 Singapore (SG)
(74) Representative: Grünecker Patent- und Rechtsanwälte PartG mbB

(57) **Abstract**

A data processing device (101) for a medical observation apparatus (102) for observing an object (106) under non-white light illumination (108) is configured to access (110) a first and second color input image (112a, 112b) representing the object (106) under non-white light illumination (108), wherein the first color input image (112a) is recorded in a first spectrum (114) and the second color input image (112b) is recorded in a second spectrum (116) different from the first spectrum (114), to access (118) a third color input image (112c) representing the object (106) under white light illumination (120), and to determine (122), based on the first, second and third color input image (112a, 112b, 112c), a color transformation function (124) that maps colors (126a, 126b) of corresponding regions (128a, 128b) in the first and second color input image (112a, 112b) onto a color (126c) of a corresponding region (128c) in the third color input image (112c). Advantageously, the third color input image (112c) requiring white light illumination needs to be taken only once and does not have to be repeated once the color transformation function is determined. The invention further relates to a medical observation apparatus (102), a computer-implemented method and an observation method.

## Description

The invention relates to a data processing device for a medical observation apparatus, such as a microscope or an endoscope, and to a method for observing an object under non-white light illumination. The invention further relates to a medical observation apparatus, and to a computer-implemented method for using, in particular for calibrating a medical observation apparatus. Moreover, the invention relates to a computer program product and computer-readable medium comprising instructions for carrying out the computer-implemented method.

In certain medical applications and other scientific scenarios, specific parts of organic matter and objects are selectively stained with fluorophores or naturally contain fluorophores. For example in surgery, the fluorescence emitted by these fluorophores can mark specific tissues to be more easily recognizable to a surgeon. In particular, a fluorophore may thus mark a tumor, which is to be surgically excised, while another fluorophore may mark blood vessels, which are to be avoided during this surgery. As an example, 5-ALA/PpIX is used as such a fluorophore for marking tumorous tissue.

The emitted fluorescence of most fluorophores is weak and not easily visible to the human eye, such that it needs to be captured in specialized images and/or by special equipment. The fluorescence images are viewed by the surgeon e.g., in a medical observation apparatus, such as a microscope or an endoscope. Triggering fluorescence of the fluorophores, however, requires illumination of the object with a fluorescence excitation spectrum, which is usually not white-light. Thus, the illumination that is required for obtaining a fluorescence image often introduces unnaturally looking colors onto the object.

At the same time, it is preferable that images shown to the surgeon appear, as natural and life-like as possible, in order to provide the surgeon with better orientation and visually familiar conditions. In particular, information about the surrounding anatomy of the object should be provided to the surgeon with as much true-color fidelity as possible.

In known approaches, the fluorescence images are supplemented by additional reflectance images that are taken under white-light illumination and provide the surgeon with a natural looking image of the surrounding area of the fluorescing fluorophore. However, this approach requires the object to be alternately or simultaneously under white light illumination and non-white light illumination. Hence, this approach results in increased effort and uses excessive time energy, data band-width as well as memory capacity.

Therefore, a need exists to easily and efficiently provide images with high anatomy rendition through non-white light illumination, which also exhibit improved color accuracy.

This need is addressed by a data processing device for a medical observation apparatus, such as a microscope or an endoscope, for observing an object under non-white light illumination, wherein the data processing device is configured to access a first and a second digital color input image representing the object under non-white light illumination, wherein the first digital color input image is recorded in a first input spectrum and the second digital color input image is recorded in a second input spectrum that is different from the first input spectrum, to access a third digital color input image representing the object under white light illumination, and to determine, based on at least part of the first, the second and the third digital color input image, a color transformation function that maps colors of corresponding regions in the first and the second digital color input image onto a color of a corresponding region in the third digital color input image.

Herein, non-white light illumination can be illumination by a non-white light source e.g., a non-standard illuminant. Likewise, white light illumination can be illumination by a white light source, in particular a standard illuminant. The first and second input spectrum can be the responsivity spectrum of one or more cameras of the medical observation apparatus used for recording the first and second digital color input image, respectively. The color mapping can be done by assigning values, sets or elements from the first and the second digital color input image in a mathematical correspondence to values, sets or elements from the third digital color input image.

The above data processing device is advantageous for the following reasons:
The color transformation function represents a correlation between the first and the second digital color input image with respect to the third digital color input image in terms of colors of corresponding regions. Herein, parts of the images that represent the same location on the object are termed "corresponding regions" as is usual in the art. These regions may comprise single pixels or pixel groups, respectively. Corresponding pixels are pixels in two images of an object that represent the same location of the object, preferably at the same scale and in the same perspective. Corresponding pixels may be located at the same position within the images, if the two images are registered. Otherwise, corresponding pixels are determined by features analysis and located at the same position of the imaged feature.

Once the color transformation function is determined using the first, the second and the third digital color input image, the color transformation function can be readily utilized to obtain a digital output image. That is, by applying the color transformation function, any two digital color input images that were taken under non-white light illumination can be converted into said digital output image which, in turn, has a true-color appearance comparable to an image taken under white light illumination.

Thereby, the first, the second and the third digital color input image serve as calibration images, which are respectively acquired for example with the medical observation apparatus. In particular, the third digital color input image requiring white light illumination needs to be taken only once and does not have to be repeated as soon as the color transformation function is determined. This allows the data processing device to save effort as well as time, energy data band-width and memory capacity during operation of the medical observation apparatus.

The above-mentioned need is further addressed by a medical observation apparatus for observing an object under non-white light illumination, wherein the medical observation apparatus comprises a data processing device according to an embodiment of the present invention, a non-white light source for illuminating the object, and a digital image recording device for recording images in the first and the second input spectrum.

The medical observation apparatus benefits from the functionality and advantages of the data processing device. In particular, the medical observation apparatus is capable of performing the above-mentioned calibration, which later during its operation saves time, energy data band-width and memory capacity.

The above need is further addressed by a computer-implemented method for using, in particular calibrating a medical observation apparatus to observe an object under non-white light illumination, the computer-implemented method comprising the steps of: accessing a first and a second digital color input image representing the object under non-white light illumination, wherein the first digital color input image is recorded in a first input spectrum and the second digital color input image is recorded in a second input spectrum that is different from the first input spectrum, accessing a third digital color input image representing the object under white light illumination, and determining, based on at least part of the first, the second and the third digital color input image, a color transformation function that maps colors of corresponding regions in the first and the second digital color input image onto a color of a corresponding region in the third digital color input image.

Advantageously, this computer-implemented method allows the same functionality and advantages as the data processing device to be implemented on a general-purpose computer, such as a PC. Accordingly, a computer-readable medium as well as a computer program product each comprising instructions, which, when executed by a computer, cause the computer to carry out the method also address the above need.

Moreover, a method for observing an object under non-white light illumination likewise addresses the above need, the method comprising the steps of the above computer-implemented method and further the steps of recording a fourth and a fifth digital color input image representing the object under non-white light illumination, the fourth digital color input image being recorded in the first input spectrum, the fifth digital color input image being recorded in the second input spectrum, accessing the color transformation function, and applying the color transformation function onto the fourth and the fifth digital color input image in order to obtain a digital output image representative of an approximation of a white light image of the object.

The above observation method yields the digital output image with anatomically accurate, white-light-image-like, true-color appearance without requiring much effort, time, energy data band-width or memory capacity.

The above data processing device, the medical observation apparatus as well as the computer-implemented method and the observation method may further be improved by any of the features, which are described in the following. The features may be used in any combination. Each of the following features is independent of the other features and advantageous on its own. Further, each of the following features may be used for improving the data processing device, the medical observation apparatus, the observation method and/or the computer-implemented method, independent of whether a feature has been described solely within the context of a device or solely in the context of a method. For example, a feature, which is described as a process step, may be used for improving the data processing device simply in that the data processing device is configured to perform this process step. Similarly, a feature of the data processing device may be used for improving e.g., the computer-implemented method in that the function performed by this feature is used as a processing step.

According to one possible embodiment, the data processing device may comprise a calibration mode and an observation mode. In the calibration mode, the data processing device is configured to carry out the steps of the computer-implemented method. In the observation mode, the data processing device is configured to access a fourth and a fifth digital color input image representing the object under non-white light illumination, the fourth digital color input image being recorded in the first input spectrum, the fifth digital color input image being recorded in the second input spectrum, to access the color transformation function calculated previously in the calibration mode, and to apply the color transformation function onto the fourth and the fifth digital color input image in order to obtain a digital output image representative of an approximation of a white light image of the object with anatomically accurate, white-light-image-like, true-color appearance. In this embodiment, the data processing device is not only capable of providing the color transformation function, but it can also carry out the steps required for the above observation method. Likewise, the medical observation apparatus may also comprise the calibration mode and observation mode.

Preferably, the medical observation apparatus and its data processing device are configured to be switched between the calibration mode and the observation mode. In particular, this switching may take place depending on a user selection signal thereby adding functional flexibility. Alternatively, the medical observation apparatus and its data processing device may be configured to start operation in the calibration mode by default after turning on and automatically switching to the observation mode after the color transformation function is successfully obtained. This may save time during emergency surgery, since unnecessary interaction with the user is omitted or at least reduced.

For verification purposes, it may be convenient to record the same object with the digital color input images of the calibration mode and of the observation mode. Thereby, it may be confirmed whether the digital output image accurately resembles the third digital input image. After this optional verification step and during normal use of the medical observation apparatus, the digital color input images of the observation mode can show different objects.

Optionally, the digital output image may be generated in real time in the observation mode. Thus, if a time-sequence of digital color input image sets is generated, e.g. by a frame rate of at least one camera, the generation of the digital output image takes preferably less time than the time between two subsequent digital color input image sets.

Each of the first, the second, the third, the fourth and the fifth digital color input image as well as the digital output image may comprise a plurality of pixels and each pixel may comprise a set of color space coordinates. With these color space coordinates and possibly other color parameters, such as hue, intensity, saturation, the color of each pixel may be represented in a color space. Such a color space may be, for example, an RGB color space. Each color space coordinate may represent a different color, in particular a different primary color. In an RGB color space, there are, for example, three color space coordinates, also termed color bands, R, G, B, which represent the red (R) the green (G) and the blue (B) primary color or color band. There are different RGB color spaces, all depending on the RGB color model. Other color spaces, such as HSV, Cl ELAB, CIEXYZ or NCS color spaces may be used instead of RGB color spaces.

The digital output image is preferably represented in the same color space as the third digital color input image. Further, the digital output image may be of the same or of a different format than the third digital color input image. It may contain the same or a different number of pixels. The same holds true if there is more than one digital color input image. In this case, the digital color input images may have the same format and/or size or be of different sizes and/or formats. Independent thereof, the digital color input images may be represented in the same color space or in different color spaces.

Optionally, the fourth and the fifth digital color input image may be combined to form an intermediate color image to which the color transformation function is applied either pixel-by-pixel or to the entire image. This combination may comprise forming a union set of the set of color space coordinates of corresponding pixels of the fourth and the fifth digital color input image.

According to another possible embodiment of the data processing device, the first and the second digital color input image may each contain a fluorescence emission signal that is representative of fluorescence emitted by at least one fluorophore in the object. This may be a fluorophore that is naturally present in the object and/or a fluorophore that has been introduced into the object. For example, the at least one fluorophore may be a certain type of material that is naturally present in the object and exhibits (auto)fluorescence if excited with the proper fluorescence excitation spectrum. As another example, the at least one fluorophore may be a fluorophore such as ICG and/or 5-ALA, which may have been injected into a patient or applied to a surgical wound. Thus, the data processing device, medical observation apparatus as well as the computer-implemented method and observation method can be used in applications involving fluorescence images.

In particular, the first digital color input image may contain a first fluorescence emission signal representative of fluorescence emitted by a first fluorophore in the object. Likewise, the second digital color input image may contain a second fluorescence emission signal that is representative of fluorescence emitted by a second fluorophore in the object. The first and/or the second fluorescence emission signal may comprise light comprising and/or consisting of light in the red color band. The red color band range may comprise or consist of light of wavelengths between about 650nm to about 700 or 750nm.

Accordingly, the non-white light illumination can be blue light, green light or any other light containing the above-mentioned suitable fluorescence excitation spectrum of the at least one fluorophore in the object, in particular, the first and/or the second fluorophore. For example, the non-white light illumination may emit light with a pre-defined excitation wavelength corresponding to the fluorescence excitation spectrum of the at least one fluorophore. The fluorescence excitation spectrum may be in the visible blue and/or green color band. The blue color band may comprise or consist of light having wavelengths between around 450nm and about 490nm. The green color band may consist of or comprise light having wavelengths between about 490nm and about 570nm. Preferably, the wavelengths in the fluorescence excitation spectrum are between about 400nm and about 480nm, more preferably between about 420nm and about 450nm.

Another particular example of a non-white light illumination may be a narrow-band illumination in the NIR (near infrared) range. The NIR range may extend in one embodiment from about 700nm to about 1100nm, more specifically from about 750nm to about 800nm. In another embodiment, it may comprise IR-A and IR-B and extend from about 700 nm to 3000 nm.

Preferably, the data processing device is configured to calculate the color transformation function as a linear transformation by solving a system of linear equations. This embodiment is simple to implement on a calculatory level. Optionally, the color transformation function may be expressed as a color transformation matrix. The color transformation matrix preferably has two dimensions. Further, the color transformation matrix may result as the product of an inverted input matrix and a calibration matrix, wherein the input matrix is representative of the first and the second digital color input image, and the calibration matrix is representative of the third digital color input image. That is, the color space coordinates from selected pixels or all pixels of each digital color input image may fill the corresponding matrix in an element-wise manner according to the location of the respective pixels.

Optionally, the color transformation function may be stored after calculation in the data processing device itself or externally. Additionally, the data processing device may have stored therein factory settings that contain a default color transformation function. Thus, the medical observation apparatus does not necessarily need to comprise a white light source for illuminating the object. Instead, the medical observation apparatus may also readily work using the default color transformation function. In applications where the default color transformation function does not render satisfactory results, an external white light source may be used for the calibration of the medical observation apparatus, in particular for the recording of the third digital color input image.

Of course, it is conceivable that the medical observation apparatus comprises a white light source and is configured to record the third digital color input image with the digital image recording device. Thereby, the medical observation apparatus is capable of recording the third digital color input image whenever necessary.

Preferably, the non-white light source and the white light source of the medical observation apparatus are both part of a switchable illumination device. For example, this switchable illumination device may have two modes for emitting either the non-white light or the white light. In particular, the switchable illumination device may comprise two different light sources or one light source and a tunable filter.

Optionally, the image recording device may comprise two cameras with different responsivity spectra that are adapted to record the first and the second digital color input image, respectively. In particular, a first camera may be provided for recording the first digital color input image and a second camera may be provided for recording the second digital color input image. Herein, the first camera may also be utilized to record the fourth digital color input image, while the second camera may be utilized for recording the fifth digital color input image. Thereby, each of the two cameras may be optimized for recording in the respective input spectrum.

According to another possible embodiment, the medical observation apparatus may be configured to record the first, the second and the third digital color input image simultaneously. In this case, it is preferable to provide the two cameras with complementary responsivity spectra. In other words, the first and the second input spectrum may be complementary to each other. In particular, the complementary responsivity spectra of the two cameras, i.e. the first and the second input spectrum may jointly spread over the entire visible spectrum. This way, the two cameras can be configured to jointly record the third digital color input image e.g., in the calibration mode. Namely, the third digital color input image may be recorded with the two cameras by adding the signals/readings of both cameras. Since the combined input spectrum of the two cameras spreads over the entire visible spectrum, the resulting image is equivalent to an image recorded by a camera with a single input spectrum spreading over the entire visible spectrum.

Alternatively, the recording of the first, the second and the third digital color input image may take place consecutively in the medical observation apparatus. Further, the digital image recording device may optionally comprise a third camera dedicated to recording the third digital color input image e.g., in the calibration mode. The input spectrum of the third camera then needs to spread over the entire visible spectrum. Accordingly, the above observation method may comprise the optional step of recording the first, the second and the third digital color input image simultaneously or consecutively.

According to another possible embodiment, the data processing device may be configured to identify fluorescent regions of the first and the second digital color input image that contain the fluorescence emission signal, in particular the first and/or the second fluorescence emission signal. In this context, fluorescent regions may be parts of an image that are contained in the fluorescence emission spectrum. This identification may be based on a threshold comparison, a color comparison and/or spectral unmixing as will be described below. In this embodiment, the data processing device is capable of discerning fluorescent regions from the rest of the first and the second digital color input image.

Moreover, the data processing device may be configured to determine, in particular calculate the color transformation function only based on regions of the first and the second digital color input image that are not identified as fluorescent regions. In other words, the data processing device may be configured to conduct a segmentation or selection within the first and the second digital color input image, which serves to cut-out/excise/ignore fluorescent parts during the calculation of the color transformation function. Optionally, corresponding parts may also be cut-out/excised/ignored from the third digital color input image during the calculation of the color transformation function.

This is advantageous, since only those regions that do not contain the fluorescence emission signal, e.g. surrounding anatomy of a tumor, require the color transformation. That is, the fluorescent regions, e.g. the tumor itself, should not appear in life-like colors, but in pseudo-colors instead. In other words, the fluorescent parts are not to be visualized in true-colors, but in prominent false-colors. This helps the surgeon recognize the tumor more easily.

Additionally or alternatively, the medical observation apparatus may comprise a graphical user interface configured to allow the user a manual pre-selection of the part of the first, the second and the third digital color input image on which the calculation of the color transformation function is to be based.

Optionally, the first, the second and the third digital color input image may be registered, preferably in a pixel-synchronized manner. Moreover, the first, the second and the third digital color input image may be converted into the same size and/or format if necessary. Besides, the first, the second and the third digital color input image may all have been recorded with the same field of view, the same magnification and/or the same perspective.

Further, the data processing device may be configured to calculate the color transformation function based on a pixel-by-pixel correlation between the first and the second digital color input image on one hand, and the third digital color input image on the other. Alternatively, the data processing device may be configured to apply a pattern recognition onto the first, the second and the third digital color input image in order to identify correlating pixels for the calculation of the color transformation function. In this case, the first, the second and the third digital color input image do not need to be registered necessarily.

According to another possible embodiment, the first and/or the second digital color input image may also contain a fluorescence excitation reflectance signal which is representative of light in the fluorescence excitation spectrum of the at least one fluorophore reflected by/off the object. In particular, the first and the second digital color input image may each be non-white light images of the object acquired with the medical observation apparatus under non-white light illumination showing fluorescence, in particular autofluorescence, and reflectance. In contrast to this, the third digital color input image, i.e. the other calibration image of the object may be acquired with the medical observation apparatus under white light illumination mainly showing reflectance.

Optionally, the two cameras may be adapted to record the first and the second digital color input image each as a multispectral or hyperspectral image of the object. Likewise, the fourth and the fifth digital color input image may each be recorded as a multispectral or hyperspectral image of the object. This brings about the advantage that the resulting additional color channels improve the accuracy of the calculated color transformation function. In contrast to this, the third digital input image as well as the digital output image have only three channels, which is sufficient for viewing by the user (e.g. surgeon).

If the multispectral or hyperspectral images are available, the data processing device may be preferably configured to perform the above-mentioned identification of the fluorescent regions by spectral unmixing. Spectral unmixing is an analysis method of multispectral or hyperspectral image pixels, known from satellite imaging. The result of spectral unmixing is a data set containing a collection of endmembers, or constituent spectra, and a set of corresponding fractions, or abundances, that indicate the proportion of each endmember present in the analyzed pixel. In other words, one can tell what substances are present in the location represented by the analyzed pixel and in what quantity relative to the other present substances.

In other words, each mixed pixel in the multispectral or hyperspectral images may be a mixture of several spectral materials in a scene. The purpose of spectral unmixing is to identify the constituent spectra from the mixed pixels and calculate the proportion of each constituent spectra in the mixed pixels in order to quantitatively decompose or "unmix" it.

The above-mentioned mixed pixels are a mixture of more than one distinct substance appearing in a single pixel, and they exist for one of two reasons. First, if the spatial resolution of a sensor is low enough that disparate materials can jointly occupy a single pixel, the resulting spectral measurement will be some composite of the individual spectra. Second, mixed pixels can result when distinct materials are combined into a homogeneous mixture. This circumstance can occur independent of the spatial resolution of the sensor.

The endmembers normally correspond to familiar macroscopic or microscopic objects, such as blood vessels, skin tissue, cancerous tissue etc. For the identification of these endmembers, known reference spectra may be used for the spectral unmixing. Consequently, as a result of the spectral unmixing, constituent spectra corresponding to macroscopic or microscopic objects visible in the multispectral or hyperspectral images are obtained. Further, fractions assigned to the constituent spectra, and associated with the analyzed pixels are obtained. Each of the fractions of a pixel indicates the proportion of a constituent spectrum contributing to the data value of the pixel. Hence, spectral unmixing, when applied to a fluorescence image, allows to distinguish multiple fluorophore signatures originating from the same source location (in case of a digital image, from the same pixel).

According to another possible embodiment, the medical observation apparatus may be configured to carry out, preferably automatically the above observation method in vivo. Preferably, in the above observation method, the fourth and the fifth digital color input image are recorded with the medical observation apparatus as well. Further, the color transformation function used in the observation method may be calculated with the computer-implemented method and/or the data processing device.

In the following, the invention is described exemplarily with reference to various examples and with reference to the drawings. The combination of features that are shown in the embodiments is not to be considered as limiting. For example, features of the embodiments, which have a technical effect as e.g. explained above, that is not needed in a specific application, may be omitted. Conversely, features described above that are not part of an embodiment described below may be added if the technical effect associated with this particular feature is needed in a specific application.

Throughout the description and the drawings, the same reference numerals are used for elements that correspond to each other with respect to function and/or structure.

In the drawings,
- Fig. 1: shows a schematic representation of a medical observation apparatus with a data processing device according to an exemplary embodiment of the present invention;
- Fig. 2: shows a schematic representation of an exemplary first, second and third digital color input image together with a schematic graphical representation of computation steps;
- Fig. 3: shows a schematic representation of an exemplary fourth and fifth digital color input image as well as an exemplary digital output image and a schematic graphical representation of computation steps;
- Fig. 4: shows a schematic representation of the medical observation apparatus with the data processing device according to another exemplary embodiment;
- Fig. 5: shows a schematic representation of a flowchart for a computer-implemented method and an observation method according to an exemplary embodiment of the present invention; and
- Fig. 6: shows a schematic representation of the medical observation apparatus with the data processing device according to another exemplary embodiment.

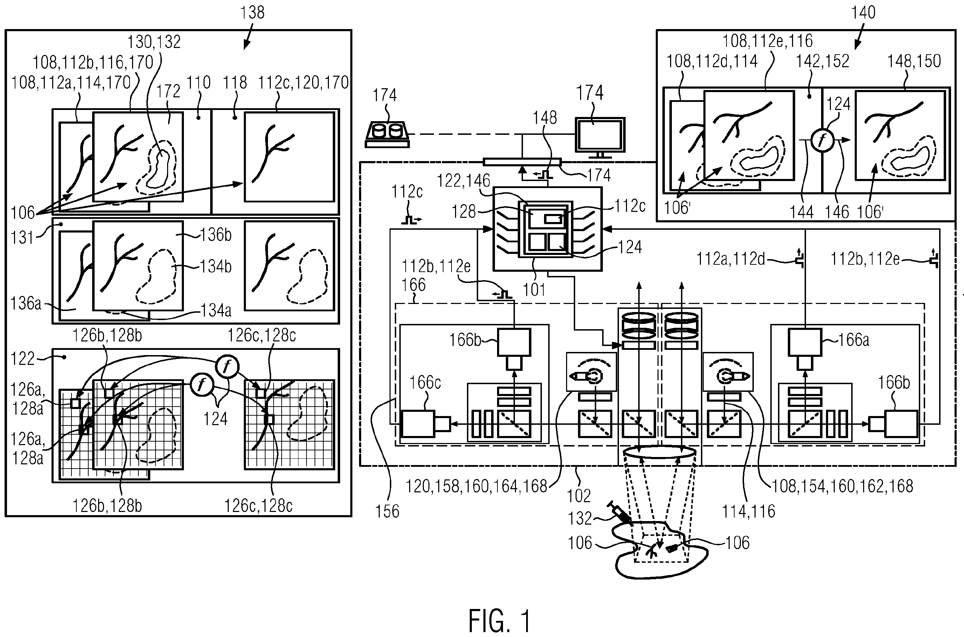

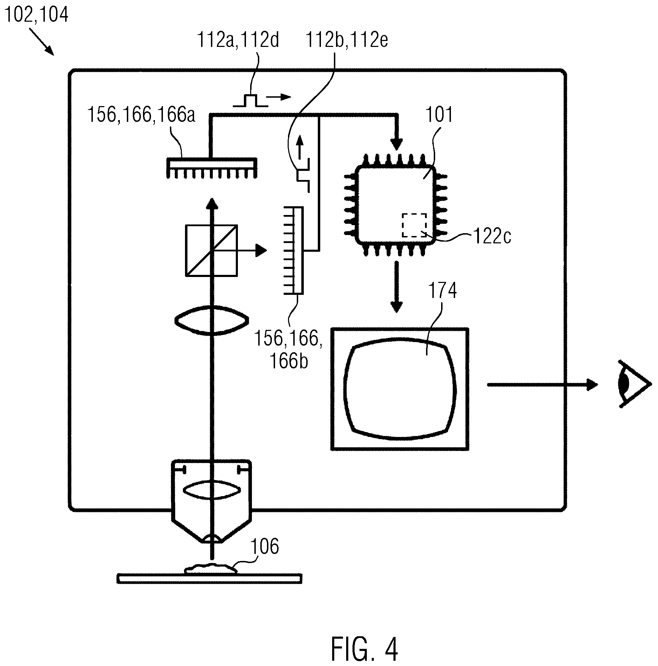

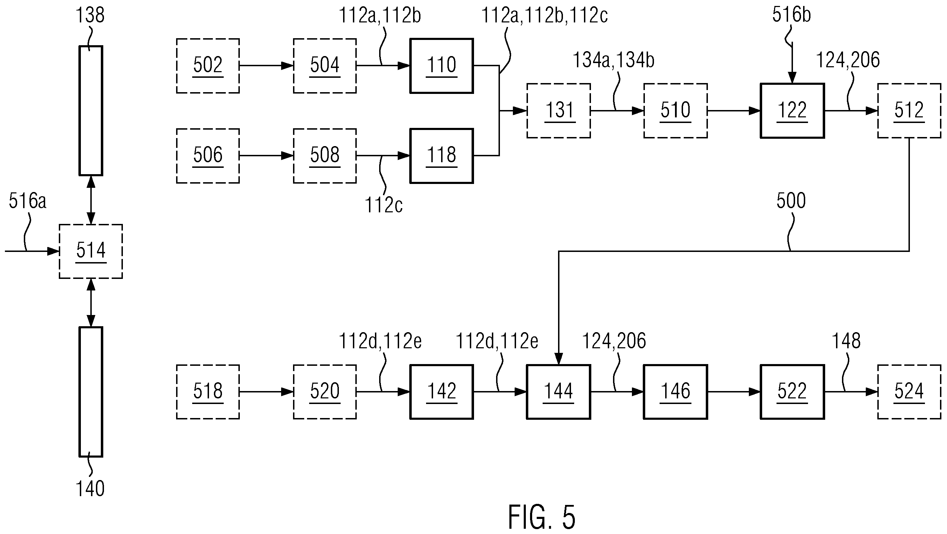

First, the structure and functionality of a data processing device 101 as well as of a medical observation apparatus 102 is explained with reference to Figs. 1 to 6. Thereafter, a computer-implemented method and a method for observing an object 106 are described with reference to Fig. 5.

The data processing device 101 may functionally be part of the medical observation apparatus 102, such as a microscope 104, endoscope (not shown), scanner, camera or any other type of scientific observation device. In particular, the data processing device 101 may be integrated in the medical observation apparatus 102 as an embedded processor or as part of such embedded processor. Further, the medical observation apparatus 102 may comprise a digital image recording device 156 for recording digital color input images 112a, 112b, 112c, 112d, 112e.

Some of these digital color input images 112a, 112b, 112c may be used for automatically calibrating the medical observation apparatus 102 and its data processing device 101. This will be explained next with reference to a calibration mode 138 of the data processing device 101. Other digital color input images 112d, 112e are used during normal operation of the medical observation apparatus 102 and its data processing device 101. That will be explained further below with reference to an observation mode 140 of the data processing device 101.

The overall purpose of the medical observation apparatus 102 and its data processing device 101 is to provide with high anatomy rendition and improved color accuracy a digital output image 148 of the object 106 under non-white light illumination 108. For this purpose, the medical observation apparatus 102 and its data processing device 101 may comprises the calibration mode 138 and the observation mode 140 mentioned above. Preferably, a switching between the calibration mode 138 and the observation mode 140 is possible based on a user selection signal. Alternatively, the medical observation apparatus 102 and its data processing device 101 may be configured to start operation in the calibration mode 138 by default after every turn-on and automatically switch to the observation mode 140 when the calibration is completed.

Preferably in the calibration mode 138, the data processing device 101 is configured to access a first digital color input image 112 and a second digital color input image 112 each representing the object 106 under non-white light illumination 108, wherein the first digital color input image 112 is recorded in a first input spectrum 114 and the second digital color input image 112 is recorded in a second input spectrum 116 that is different from the first input spectrum 114. Further, the data processing device 101 is configured to access a third digital color input image 112 representing the object 106 under white light illumination 120.

Herein, non-white light illumination 108 can be illumination by a non-white light source 154 e.g., a non-standard illuminant 162. Likewise, white light illumination 120 can be illumination by a white light source 158, in particular a standard illuminant 164. The first input spectrum 114 and the second input spectrum 116 can each be the responsivity spectrum of one or more cameras166 of the medical observation apparatus 102, in particular the digital image recording device 156.

As can be seen in Fig. 1, the medical observation apparatus 102 comprises the non-white light source 154 for illuminating the object 106. Further, the medical observation apparatus 102 may comprise the white light source 158 for illuminating the object 106 as well. Preferably, the non-white light source 154 and the white light source 158 of the medical observation apparatus 102 are both part of a switchable illumination device 160. For example, this switchable illumination device 160 may have two modes for emitting either the non-white light or the white light. In particular, the switchable illumination device 160 may comprise two different light sources 168 or one light source and a tunable filter (not shown).

Optionally, the digital image recording device 156 may comprise two cameras 166 with different responsivity spectra that are adapted to record the first and the second digital color input image 112a, 112b, respectively. In particular, a first camera 166a may be provided for recording the first digital color input image 112a and a second camera 166b may be provided for recording the second digital color input image 112b. Herein, the first camera 166a may also be utilized to record the fourth digital color input image 112d, while the second camera 166b may be utilized for recording the fifth digital color input image 112e.

It is convenient to provide the two cameras 166a, 166b with complementary responsivity spectra. In other words, the first and the second input spectrum 114, 116 may be complementary to each other. In particular, the complementary responsivity spectra of the two cameras 166a, 166b, i.e. the first and the second input spectrum 114, 116 may jointly spread over the entire visible spectrum. This way, the two cameras 166a, 166b can be configured to jointly record the third digital color input image 112c e.g., in the calibration mode 138. Namely, the third digital input image 112c may be recorded with the two cameras 166a, 166b by adding the signals/readings of both cameras. Since the combined input spectrum of the two cameras166a, 166b spreads over the entire visible spectrum, the resulting third digital color input image 112e is equivalent to an image recorded by a camera with an input spectrum spreading over the entire visible spectrum. Alternatively, the image recording device 156 may comprise a third camera 166c dedicated to recording the third digital color input image 112c. The input spectrum of this third camera 166c then needs to spread over the entire visible spectrum.

Returning back to the functionality of the data processing device 101: the data processing device 101 is configured to determine, based on at least part of the first, the second and the third digital color input image 112a, 112b, 112c, a color transformation function 124 that maps colors 126a, 126b of corresponding regions 128a, 128b in the first and the second digital color input image 112a, 112b onto a color 126c of a corresponding region 128c in the third digital color input image 112c. This color mapping can be done by assigning values, sets or elements from the first and the second digital color input image 112a, 112b in a mathematical correspondence to values, sets or elements from the third digital color input image 112c.

Hence, the color transformation function 124 represents a correlation between the first and the second digital color input image 112a, 112b with respect to the third digital color input image 112c in terms of the colors 126a, 126b, 126c of the corresponding regions 128a, 128b, 128c. Herein, parts of the images 112a, 112b, 112c that represent the same location 216a, 216b 216c on the object 106 are termed "corresponding regions" as is usual in the art. These regions may comprise single pixels or pixel groups, respectively. In general, corresponding pixels are pixels in two images of an object that represent the same location of the object, preferably at the same scale and in the same perspective. Corresponding pixels may be located at the same position within the images, if the two images are registered. Otherwise, corresponding pixels are determined by features analysis and located at the same position of the imaged feature.

As already mentioned, the first, the second and the third digital color input image 112a, 112b, 112c may be registered, preferably in a pixel-synchronized manner. Moreover, the first, the second and the third digital color input image 112a, 112b, 112c may be converted into the same size and/or format if necessary. Besides, the first, the second and the third digital color input image 112a, 112b, 112c may all have been recorded with the same field of view, the same magnification and/or the same perspective.

In the calibration mode 138, the first, the second and the third digital color input image 112a, 112b, 112c are preferably acquired with the medical observation apparatus 102 and serve as calibration images 170. Especially the third digital color input image 112c requiring white light illumination 120 needs to be taken only once and does not have to be repeated as soon as the color transformation function 124 is determined successfully.

Once the color transformation function 124 is determined, it can be readily utilized to obtain the digital output image 148 in the observation mode 140. That is, the data processing device 101 may be configured, in the observation mode 140, to access a fourth digital color input image 112 and a fifth digital color input image 112 each representing the object 106 (or another object 106') under non-white light illumination 108. Similar to the first digital color input image 112 from the calibration mode 138, the fourth digital color input image 112 is also recorded in the first input spectrum 114. And the fifth digital color input image 112 is recorded in the second input spectrum 116, just like the second digital color input image 112 of the calibration mode 138.

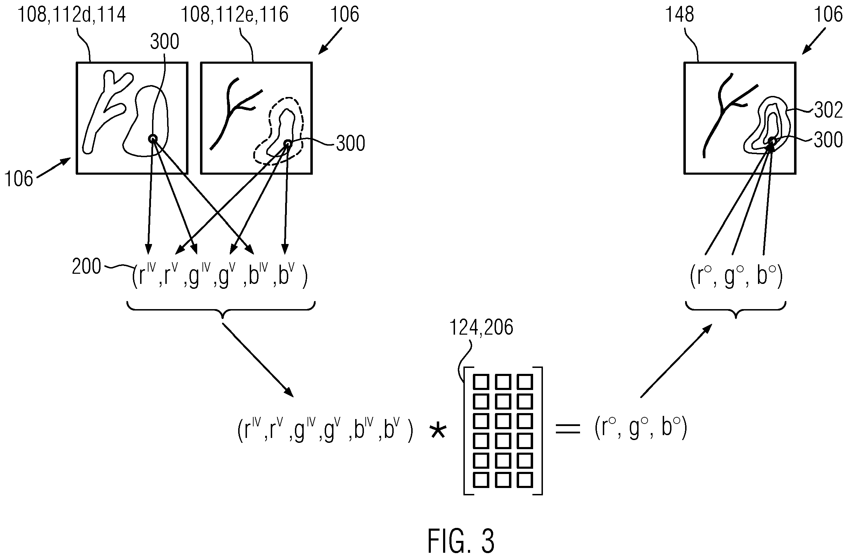

As is shown in Fig. 3, the data processing device 101 may be configured to apply the color transformation function 124 onto the fourth and the fifth digital color input image 112d, 112e in order to obtain the digital output image 148, which is representative of an approximation of a white light image of the object 106 with anatomically accurate, white-light-image-like appearance. In other words, applying the color transformation function 124 on two digital color input images that were taken under non-white light illumination 108, yields the digital output image 148, which has a true-color appearance comparable to an image taken under white light illumination 120.

Optionally, the fourth and the fifth digital color input image 112d, 112e may be combined to form an intermediate color image (not shown) to which the color transformation function 124 is applied either pixel-by-pixel or to the entire image. This combination may comprise forming a union set 200 of the set of color space coordinates 202 of corresponding pixels 300 of the fourth and the fifth digital color input image 112d, 112e.

Each of the first, the second, the third, the fourth and the fifth digital color input image 112a, 112b, 112c, 112d, 112e as well as the digital output image may comprise a plurality of pixels and each pixel may comprise a set of color space coordinates 202. With these color space coordinates 202 and possibly other color parameters, such as hue, intensity and saturation, the color of each pixel may be represented in a color space. Such a color space may be, for example, an RGB color space. Each color space coordinate may represent a different color, in particular a different primary color. In an RGB color space, there are, for example, three color space coordinates r, g, b from the color bands, R, G, B, which represent the red (r, R) the green (g, G) and the blue (b, B) primary color or color band.

The digital output image 148 is preferably represented in the same color space as the third digital color input image 112c. Further, the digital output image 148 may be of the same or of a different format than the third digital color input image 112c. It may contain the same or a different number of pixels.

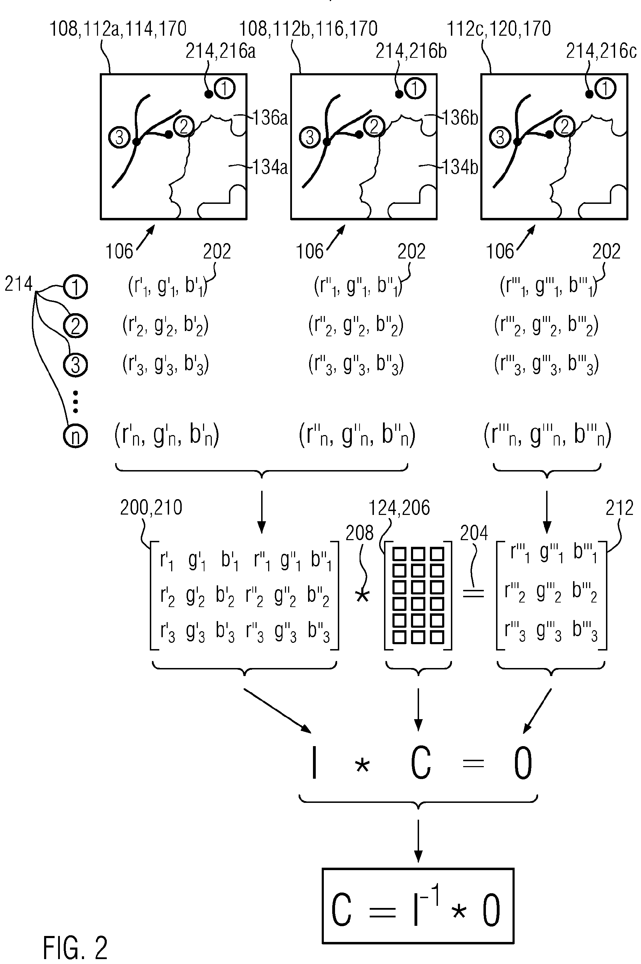

In Fig. 2, the color space coordinates r^{I}, g^{I}, b^{I} belong to the first digital color input image 112a. Analogously, the color space coordinates belonging to the second digital color input image 112b are denoted r^{II}, g^{II}, b^{II} (r^{III}, g^{III}, b^{III} → third digital color input image 112c; r^{IV},g^{IV},b^{IV} → fourth digital color input image 112d; r^{V},g^{V},b^{V} → fifth digital color input image 112e; and r^{O},g^{O},b^{O} → the digital output image). The color coordinates rₙ, gₙ, bₙ belong to the n-th pixel of the respective image.

If the calibration images 170 are registered in a pixel-synchronized manner, the data processing device 101 may be configured to calculate the color transformation function 124 based on a pixel-by-pixel correlation between the first and the second digital color input image 112a, 112b on one hand and the third digital color input image 112c on the other. Alternatively, the data processing device 101 may be configured to apply a pattern recognition onto the first, the second and the third digital color input image 112a, 112b, 112c in order to identify correlating pixels for the calculation of the color transformation function 124. In this case, the first, the second and the third digital color input image 112a, 112b, 112c do not need to be registered.

Preferably, the data processing device 101 is configured to calculate the color transformation function 124 as a linear transformation by solving a system of linear equations 204. In particular, the color transformation function 124 may be expressed as a color transformation matrix 206. The color transformation matrix 206 preferably has two dimensions. Further, the color transformation matrix 206 may result as the product 208 of an inverted input matrix 210 and a calibration matrix 212, wherein the input matrix 210 is representative of the first and the second digital color input image 112a, 112b, and the calibration matrix 212 is representative of the third digital color input image 112c. That is, the color space coordinates 202 from selected pixels 214 or all pixels of each calibration image 170 may fill the corresponding matrix 210, 212 in an element-wise manner according to the location of the respective pixels.

After calculation, the color transformation function 124 may be stored in the data processing device 101 itself or externally. Accordingly, the data processing device 101 may be configured to access the color transformation function 124 calculated in the calibration mode 138 directly or from the external storage location. Additionally, the data processing device 101 may have stored therein factory settings that contain a default color transformation function.

As can be seen in Fig. 1 and 3, the first and the second digital color input image 112a, 112b as well as the fourth and the fifth digital color input image 112d, 112e may each contain a fluorescence emission signal 130 that is representative of fluorescence emitted by at least one fluorophore 132 in the object 106. This may be a fluorophore that is naturally present in the object 106 and/or a fluorophore that has been introduced into the object 106. For example, the at least one fluorophore 132 may be a certain type of material that is naturally present in the object 106 and exhibits (auto)fluorescence if excited with the proper fluorescence excitation spectrum. As another example, the at least one fluorophore 132 may be a fluorophore such as ICG and/or 5-ALA, which may have been injected into a patient or applied to a surgical wound.

Additionally, the first and the second digital color input image 112a, 112b as well as the fourth and fifth digital color input image 112d, 112e may also contain a fluorescence excitation reflectance signal 516a which is representative of light in the fluorescence excitation spectrum of the at least one fluorophore 132 reflected by/off the object 106. In particular, the first and the second digital color input image 112a, 112b may each be non-white light images of the object 106 acquired with the medical observation apparatus 102 under the non-white light illumination showing fluorescence, in particular autofluorescence, and reflectance. In contrast to this, the third digital color input image 112c, i.e. the actual calibration image 170 of the object 106 may be acquired with the medical observation apparatus 102 under white light illumination 120 mainly showing reflectance.

Accordingly, the non-white light illumination 108 can be blue light, green light or any other light containing the above-mentioned proper fluorescence excitation spectrum of the at least one fluorophore 132 in the object 106. For example, the non-white light illumination 108 may emit light with a pre-defined excitation wavelength corresponding to the fluorescence excitation spectrum of the at least one fluorophore 132. The fluorescence excitation spectrum may be in the visible blue and/or green color band. The blue color band may comprise or consist of light having wavelengths between around 450nm and about 490nm. The green color band may consist of or comprise light having wavelengths between about 490nm and about 570nm. Preferably, the wavelengths in the fluorescence excitation spectrum are between about 400nm and about 480nm, more preferably between about 420nm and about 450nm.

Another particular example of a non-white light illumination 108 may be a narrow-band illumination in the NIR (near infrared) range. The NIR range may extend in one embodiment from about 700nm to about 1100nm, more specifically from about 750nm to about 800nm. In another embodiment, it may comprise IR-A and IR-B and extend from about 700 nm to 3000 nm.

In the calibration mode 138, the data processing device 101 may be configured to identify fluorescent regions 134a, 134b of the first and the second digital color input image 112a, 112b that contain the fluorescence emission signal 130. In this context, fluorescent regions 134a, 134b may be parts of an image that are contained in the fluorescence emission spectrum. The identification of such regions may be based on a threshold comparison, a color comparison and/or spectral unmixing as will be described below.

Moreover, the data processing device 101 may be configured to determine, in particular calculate the color transformation function 124 only based on regions of the first and the second digital color input image 112a, 112b that are not identified as fluorescent regions 134a, 134b. In other words, the data processing device 101 may be configured to conduct a segmentation or selection within the first and the second digital color input image 112a, 112b, which serves to cut-out/excise/ignore fluorescent parts during the calculation of the color transformation function 124. Optionally, corresponding parts may also be cut-out/excised/ignored from the third digital color input image 112c during the calculation of the color transformation function 124. This is shown in Fig. 1.

By excising the fluorescent regions 134a, 134b, it is safeguarded that only those regions that do not contain the fluorescence emission signal, e.g. the surrounding anatomy of a tumor, receive the color transformation. As a result, the fluorescent regions 134a, 134b, e.g. the tumor itself, will not appear in life-like colors in the digital output image 148, but in pseudo-colors 302 instead. In other words, the fluorescent parts will not be visualized in true-colors, but in prominent false-colors. This is shown in Fig. 1 and helps the surgeon recognized the tumor more easily.

Optionally, the two cameras 166a, 166b may be adapted to record the first and the second digital color input image 112a, 112b each as a multispectral or hyperspectral image (not shown) of the object 106. Likewise, the fourth and the fifth digital color input image 112d, 112e may each be recorded as a multispectral or hyperspectral image (not shown) of the object 106. This brings about the advantage that the resulting additional color channels improve the accuracy of the calculated color transformation function 124. In contrast to this, the third digital input image 112c as well as the digital output image 148 have only three channels, which is sufficient for viewing by the user.

If the multispectral or hyperspectral images are available, the data processing device 101 may be preferably configured to perform the above-mentioned identification of the fluorescent regions 134a, 134b by spectral unmixing. Spectral unmixing is an analysis method of multispectral or hyperspectral image pixels, known from satellite imaging. The result of spectral unmixing is a data set containing a collection of endmembers, or constituent spectra, and a set of corresponding fractions, or abundances, that indicate the proportion of each endmember present in the analyzed pixel. In other words, one can tell what substances are present in the location represented by the analyzed pixel and in what quantity relative to the other present substances.

In other words, each mixed pixel in the multispectral or hyperspectral images may be a mixture of several spectral materials in a scene. The purpose of spectral unmixing is to identify the constituent spectra from the mixed pixels and calculate the proportion of each constituent spectra in the mixed pixels in order to quantitatively decompose or "unmix" it.

The above-mentioned mixed pixels are a mixture of more than one distinct substance appearing in a single pixel, and they exist for one of two reasons. First, if the spatial resolution of a sensor is low enough that disparate materials can jointly occupy a single pixel, the resulting spectral measurement will be some composite of the individual spectra. Second, mixed pixels can result when distinct materials are combined into a homogeneous mixture. This circumstance can occur independent of the spatial resolution of the sensor.

The endmembers normally correspond to familiar macroscopic or microscopic objects, such as blood vessels, skin tissue, cancerous tissue etc. For the identification of these endmembers, known reference spectra may be used for the spectral unmixing. Consequently, as a result of the spectral unmixing, constituent spectra corresponding to macroscopic or microscopic objects visible in the multispectral or hyperspectral images are obtained. Further, fractions assigned to the constituent spectra, and associated with pixels are obtained. Each of the fractions of a pixel indicates the proportion of a constituent spectrum contributing to the data value of the pixel. Hence, spectral unmixing, when applied to a fluorescence image, allows to distinguish multiple fluorophore signatures originating from the same source location (in case of a digital image, from the same pixel).

Fig. 5 shows a flowchart of the computer-implemented method and the observation method according to one possible embodiment. Herein, the computer-implemented method is preferably performed in the calibration mode 138 of the medial observation apparatus 102 and its data processing device 101. Likewise, the observation method is preferably performed in the observation mode 140 of the medial observation apparatus 102 and its data processing device 101. The result of the computer-implemented method, i.e. the color transformation function 124, in particular the color transformation matrix 206 may serve as an input for the observation method. This is symbolized by arrow 500 in Fig. 5.

The computer-implemented method serves mainly for calibrating the medical observation apparatus 102. As such, the computer-implemented method comprises the step 110 of accessing the first and the second digital color input image 112a, 112b representing the object 106 under non-white light illumination 108. Further, the computer-implemented method comprises the step 118 of accessing the third digital color input image 112c representing the object 106 under white light illumination.

The accessing steps 110, 118 may be optionally preceded by the step 502 of illuminating the object 106 with the non-white light source 154, the step 504 of recording the first and the second digital color input image 112a, 112b with the medical observation apparatus 102, the step 506 of illuminating the object 106 with the white light source 158 and/or the step 508 of recording the third digital color input image 112c with the medical observation apparatus 102.

Herein, the object 106 may be a pre-defined calibration gauge (not shown), e.g. a pre-defined calibration card comprising a color-printed surface with one or more color tables. The third digital color input image 112c of the calibration gauge may be stored in the data processing device 101 as part of the above-mentioned factory settings. Hence, the steps 506, 508 may be omitted and the medical observation apparatus 102 does not necessarily need to comprise any white light source for illuminating the object 106. In other words, the first and the second digital color input image 112a, 112b of the calibration gauge may be obtained through the steps 502, 504, while the third digital color input image 112c of the calibration gauge is pre-stored in the data processing device. If the computer-implemented method is carried out on a general computer as part of an image post-processing routine, the steps 502, 504 may also be omitted and the first and the second digital color input image 112a, 112b may be accessed in step 110 from an external source.

Next, follows the step 122 of determining, based on at least part of the first, the second and the third digital color input image 112a, 112b, 112c, the color transformation function 124. The concrete procedure of determining the color transformation function 124 has been explained above with respect to the color transformation matrix 206 and thus will be omitted here.

Previously, the step 131 of identifying the fluorescent regions 134a, 134b in the first and the second digital color input image 112a, 112b may be carried out. Thereafter, the step 510 of cutting out the fluorescent regions 134a, 134b from the first and the second digital color input image 112a, 112b may ensue. Optionally, a region of the third digital color input image 112c corresponding to the fluorescent regions 134a, 134b may also be cut out in step 510. This yields the part of the first, the second and the third digital color input image 112a, 112b, 112c, based on which the color transformation function 124 is determined in step 122.

Optionally, the resulting color transformation function 124 may be stored in step 512. Now, the step 514 of switching from the calibration mode 138 to the observation mode 140 may be prompted by a user selection signal 516a. Another user selection signal 516b may be used when selecting the part of the first, the second and the third digital color input image 112a, 112b, 112c, based on which the color transformation function 124 is determined in step 122.

Once the observation mode 140 is selected, the step 142 of accessing the fourth and the fifth digital color input image 112d, 112e will follow. Beforehand, the optional step 518 of illuminating the object 106 with the non-white light source 154 and the step 520 of recording the fourth and the fifth digital color input image 112d, 112e with the medical observation apparatus 102 may take place. Alternatively, if the above-mentioned image post-processing routine is performed, the steps 518, 520 may again be omitted and the fourth and the fifth digital color input image 112d, 112e may be accessed in step 142 from an external source.

Next, the step 144 of accessing the color transformation function 124 and the step 146 of applying the color transformation function 124 onto the fourth and the fifth digital color input image 112d, 112e are carried out. Thereby, the step 522 of obtaining the digital output image 148 is achieved. Optionally, the step 524 of outputting the digital output image 148 e.g., to a display device 174 of the medical observation apparatus 102 may complete the observation method. Or, the observation method may be repeated from its beginning.

According to an embodiment that is not shown in the figures, the medical observation apparatus 101 may be configured to carry out, preferably automatically the above observation method in vivo. Preferably, in the above observation method, the fourth and the fifth digital color input image 112d, 112e are recorded with the medical observation apparatus 102 as well. Further, the color transformation function 124 used in the observation method may be calculated with the computer-implemented method and/or the data processing device 101.

As used herein the term "and/or" includes any and all combinations of one or more of the associated listed items and may be abbreviated as "f".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

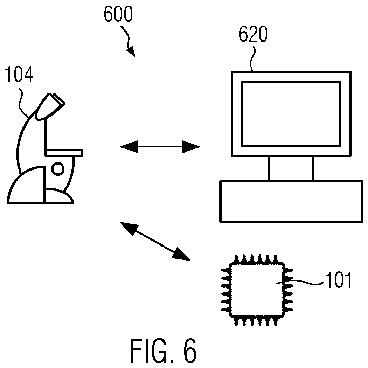

Some embodiments relate to a microscope comprising a system as described in connection with one or more of the Figs. 1 to 5. Alternatively, a microscope may be part of or connected to a system as described in connection with one or more of the Figs. 1 to 5. Fig. 6 shows a schematic illustration of a system 600 configured to perform a method described herein. The system 600 comprises a microscope 104 and a computer system 620. The microscope 104 is configured to take images and is connected to the computer system 620. The computer system 620 is configured to execute at least a part of a method described herein. The computer system 620 may also be configured to execute a machine learning algorithm. The computer system 610 and microscope 104 may be separate entities but can also be integrated together in one common housing. The computer system 620 may be part of a central processing system of the microscope 104 and/or the computer system 620 may be part of a subcomponent of the microscope 104, such as a sensor, an actor, a camera or an illumination unit, etc. of the microscope 104.

The computer system 620 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 620 may comprise any circuit or combination of circuits. In one embodiment, the computer system 620 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 620 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 620 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 620 may also include a display device, one or more speakers, and a keyboard and/or controller, which can include a mouse, trackball, touch screen, voice-recognition device, or any other device that permits a system user to input information into and receive information from the computer system 620.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

### REFERENCE NUMERALS

- 101: data processing device
- 102: medical observation apparatus
- 104: microscope
- 106, 106': object
- 108: non-white light illumination
- 110: access
- 112a: first digital color input image
- 112b: second digital color input image
- 112c: third digital color input image
- 112d: fourth digital color input image
- 112e: digital color input image
- 114: first input spectrum
- 116: second input spectrum
- 118: access
- 120: white light illumination
- 122: determine
- 124: color transformation function
- 126a, 126b, 126c: color
- 128a, 128b, 128c: region
- 130: fluorescence emission signal
- 131: identify
- 132: fluorophore
- 134a, 134b: fluorescent region
- 136a, 136b: region
- 138: calibration mode
- 140: observation mode
- 142: access
- 144: access
- 146: apply
- 148: digital output image
- 150: approximation of a white light image
- 152: record
- 154: non-white light source
- 156: digital image recording device
- 158: white light source
- 160: switchable illumination device
- 162: non-standard illuminant
- 164: standard illuminant
- 166, 166a, 166b, 166c: camera
- 168: light source
- 170: calibration image
- 172: fluorescence excitation reflectance signal
- 174: display device
- 200: union set
- 202: color space coordinate
- 204: system of linear equations
- 206: color transformation matrix
- 208: product
- 210: input matrix
- 212: calibration matrix
- 214: selected pixel
- 216a, 216b, 216c: location
- 300: corresponding pixels
- 302: pseudo-color
- 500: arrow
- 502: step
- 504: step
- 506: step
- 508: step
- 510: step
- 512: step
- 514: step
- 516a, 516b: user selection signal
- 518: step
- 520: step
- 522: step
- 524: step
- 600: system
- 620: computer system

## Claims

1. Data processing device (101) for a medical observation apparatus (102), such as a microscope (104) or an endoscope, for observing an object (106) under non-white light illumination (108), wherein the data processing device (101) is configured:
- to access (110) a first and a second digital color input image (112a, 112b) representing the object (106) under non-white light illumination (108), wherein the first digital color input image (112a) is recorded in a first input spectrum (114) and the second digital color input image (112b) is recorded in a second input spectrum (116) that is different from the first input spectrum (114),
- to access (118) a third digital color input image (112c) representing the object (106) under white light illumination (120), and
- to determine (122), based on at least part of the first, the second and the third digital color input image (112a, 112b, 112c), a color transformation function (124) that maps colors (126a, 126b) of corresponding regions (128a, 128b) in the first and the second digital color input image (112a, 112b) onto a color (126c) of a corresponding region (128c) in the third digital color input image (112c).

2. Data processing device (101) according to claim 1, wherein the first and the second digital color input image (112a, 112b) contain a fluorescence emission signal (130) that is representative of fluorescence emitted by at least one fluorophore (132) in the object (106).

3. Data processing device (101) according to claim 2, wherein the data processing device (101) is configured to identify (131) fluorescent regions (134a, 134b) of the first and the second digital color input image (112a, 112b) that contain the fluorescence emission signal (130).

4. Data processing device (101) according to claim 3, wherein the data processing device (101) is configured to determine (122) the color transformation function (124) based on regions (136a, 136b) of the first and the second digital color input image (112a, 112b) that are not identified as fluorescent regions (134a, 134b).

5. Data processing device (101) according to any one of claims 1 to 4, wherein the data processing device (101) comprises a calibration mode (138) and an observation mode (140), wherein, in the calibration mode (138), the data processing device (101) is configured according to any one of claims 1 to 4, and wherein, in the observation mode (140), the data processing device (101) is configured:
- to access (142) a fourth and a fifth digital color input image (112d, 112e) representing the object (106, 106') under non-white light illumination (108), the fourth digital color input image (112d) being recorded in the first input spectrum (114), the fifth digital color input image (112e) being recorded in the second input spectrum (116),
- to access (144) the color transformation function (124), and
- to apply (146) the color transformation function (124) onto the fourth and the fifth digital color image (112a, 112b) in order to obtain a digital output image (148) representative of an approximation of a white light image (150) of the object (106).

6. Data processing device (101) according to claim 5, wherein the data processing device (101) is configured to be switched between the calibration mode (138) and the observation mode (140).

7. Computer-implemented method for using a medical observation apparatus (102) to observe an object (106) under non-white light illumination (108), the computer-implemented method comprising the steps of:
- accessing (110) a first and a second digital color input image (112a, 112b) representing the object (106) under non-white light illumination (108), wherein the first digital color input image (112a) is recorded in a first input spectrum (114) and the second digital color input image (112b) is recorded in a second input spectrum (116) that is different from the first input spectrum (114),
- accessing (118) a third digital color input image (112c) representing the object (106) under white light illumination (120), and
- determining (122), based on at least part of the first, the second and the third digital color input image (112a, 112b, 112c), a color transformation function (124) that maps colors (126a, 126b) of corresponding regions (128a, 128b) in the first and the second digital color input image (112a, 112b) onto a color (126c) of a corresponding region (128c) in the third digital color input image (112c).

8. Method for observing an object (106) under non-white light illumination (108), the method comprising:
- the computer-implemented method of claim 7
and the steps of:
- recording (152) a fourth and a fifth digital color input image (112d, 112e) representing the object (106, 106') under non-white light illumination (108), the fourth digital color input image (112d) being recorded in the first input spectrum (114), the fifth digital color input image (112e) being recorded in the second input spectrum (116),
- accessing (144) the color transformation function (124), and
- applying (146) the color transformation function (124) onto the fourth and the fifth digital color image (112d, 112e) in order to obtain a digital output image (148) representative of an approximation of a white light image (150) of the object (106).

9. Medical observation apparatus (102) for observing an object (106) under non-white light illumination (108), wherein the medical observation apparatus (102) comprises:
- a data processing device (101) according to any one of claims 1 to 6,
- a non-white light source (154) for illuminating the object (106), and
- a digital image recording device (156) for recording images (112a, 112b, 112c, 112d, 112e) in the first and the second input spectrum (114, 116).

10. Medical observation apparatus (102) according to claim 9, wherein the medical observation apparatus (102) comprises a white light source (158) and is configured to record the third digital color input image (112c) with the digital image recording device (156).

11. Medical observation apparatus (102) according to claim 10 wherein the non-white light source (154) and the white light source (158) are both part of a switchable illumination device (160).

12. Medical observation apparatus (102) according to claim 10 or 11, wherein the medical observation apparatus (102) is configured to record the first, second and third digital color input image (112a, 112b, 112c) simultaneously or consecutively.

13. Medical observation apparatus (102) according to any one of claims 9 to 12, wherein the medical observation apparatus (102) is configured to carry out the method according to claim 8 in vivo.

14. A computer program product comprising instructions, which, when the program is executed by a computer, cause the computer to carry out the computer-implemented method of claim 7.

15. A computer-readable medium comprising instructions, which, when the program is executed by a computer, cause the computer to carry out the computer-implemented method of claim 7.
